# EUROPEAN PATENT APPLICATION

(11) **EP 2 998 390 A1**
(43) Date of publication of application: **23.03.2016**
(21) Application number: 14798397.7
(22) Date of filing: 14.05.2014
(51) Int. Cl.: C12N 5/071, A61K 35/32, A61P 25/00

(54) **METHOD FOR PRODUCING GRAFT MATERIAL FOR TREATING NERVE DAMAGE**

(30) Priority: 14.05.2013 JP 2013102426
(71) Applicant: Advanced Center For Tissue Engineering Ltd., Tokyo 104-0061 (JP)
(72) Inventor: TEZUKA, Kenichi, Gifu-shi Gifu 501-1112 (JP); KAWAGUCHI, Tomoko, Gifu-shi Gifu 501-1112 (JP); KUNISADA, Takahiro, Gifu-shi Gifu 501-1112 (JP); SHIBATA, Toshiyuki, Gifu-shi Gifu 501-1112 (JP); FUKUMITSU, Hidefumi, Gifu-shi Gifu 501-1113 (JP); FURUKAWA, Shoei, Gifu-shi Gifu 501-1113 (JP)
(74) Representative: Keirstead, Tanis Evelyne
(86) International application number: PCT/JP2014/062881
(87) International publication number: WO 2014/185470

(57) **Abstract**

An object of the present invention is to provide a method for efficiently and reproducibly producing a graft material having a high recovery effect on dysfunction caused by nerve damage. The present invention provides a method for producing a graft material for treating nerve damage, including a step of culturing a dental pulp stem cell in a medium substantially containing no growth factors except FGF2, and others.

## Description

### Technical Field

The present invention relates to a method for producing a graft material for treating nerve damage by using a dental pulp stem cell, and others.

### Background Art

The spinal cord is a path for transmitting kinetic and perceptual information between peripheral tissues and the brain. Injury of the spinal cord causes a severe physical disability such as motor paralysis and perceptual disorder. Neither effective treatment for this nor partial functional reconstruction is expected. In recent years, studies have been aggressively promoted worldwide; however a fundamental therapy has not yet been developed.

In this country, there are about 100,000 patients with spinal cord injury and about 5,000 patients have newly been injured per year. There are peaks of the number of patients at around 20 years old and around 60 years old. Spinal cord injury is caused by accidents during driving and sporting activities mainly in middle aged persons and caused by spinal fracture by application of minor impact in elderly persons, and others. A functional loss of a body is rarely recovered and the patients thereafter live a significantly limited life.

It has recently been reported that dysmobility in rats caused by spinal cord injury is significantly recovered when human dental pulp stem cells were grafted (Non Patent Literature 1). However, it is still desired to develop a method for treating nerve damage with higher reproducibility and higher recovery effect.

### Citation List

### Non Patent Literature

Non Patent Literature 1: Sakai K. et al., J Clin Invest 122: 80-90

### Summary of Invention

### Technical Problem

An object of the present invention is to provide a method for efficiently and reproducibly producing a graft material having a high recovery effect on dysfunction caused by nerve damage.

### Solution to Problem

The present inventors have intensively conducted studies with a view to attaining the above object. As a result, they found that if dental pulp stem cells are cultured in a conventional medium supplemented with FGF2 at a relatively high concentration and the resultant culture is grafted to a model with spinal cord injury, the effect of recovering motor function significantly improved compared to the conventional methods; and that the recovery effect can be obtained with good reproducibility if dental pulp stem cells having a predetermined gene expression pattern are used. Based on the findings, they accomplished the present invention.

More specifically, the present invention relates to
[1] A method for producing a graft material for treating nerve damage, comprising
   a step of culturing a dental pulp stem cell in a medium substantially containing no growth factors except FGF2,
[2] The method according to above [1], wherein the medium substantially containing no growth factors except FGF2 is a serum-containing base medium supplemented with FGF2 alone as a growth factor,
[3] The method according to above [2], wherein the serum in the medium has a concentration of less than 15 wt%,
[4] The method according to above [1], wherein the medium substantially containing no growth factors except FGF2 is a commercially available medium for culturing mesenchymal stem cells supplemented with FGF2 alone as a growth factor,
[5] The method according to any one of above [1] to [4], wherein FGF2 in the medium has a concentration of 5 ng/mL or more,
[6] The method according to above [5], wherein FGF2 in the medium has a concentration of 7 ng/mL or more,
[7] The method according to any one of above [1] to [6], wherein the dental pulp stem cell used is a dental pulp stem cell, in which the expression level of 10% or more of genes in the group of genes listed in Table 1 is 5 times or more as high as an average expression level of the genes in dental pulp stem cells,
[8] The method according to any one of above [1] to [7], wherein the dental pulp stem cell used is a dental pulp stem cell, in which the expression level of at least one gene selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as an average expression level of genes in dental pulp stem cells,
[9] The method according to any one of above [1] to [8], wherein the dental pulp stem cell used is a dental pulp stem cell, in which the expression level of 10% or more of genes in the group of genes listed in Table 2 is 5 times or more as low as an average expression level of the genes in dental pulp stem cells,
[10] The method according to any one of above [1] to [9], wherein the dental pulp stem cell used is a dental pulp stem cell, in which the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as an average expression level of genes in dental pulp stem cells,
[11] The method according to any one of above [1] to [6], wherein among two or more groups of dental pulp stem cells, a group of dental pulp stem cells in which the expression level of 10% or more of genes in the group of genes listed in Table 1 is 5 times or more as high as other groups of cells, is selected as the dental pulp stem cell to be used,
[12] The method according to any one of above [1] to [6] and [11], wherein among two or more groups of dental pulp stem cells, a dental pulp stem cell in which the expression level of at least one gene selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as other groups of cells, is selected as the dental pulp stem cell to be used,
[13] The method according to any one of above [1] to [6], and [11] and [12], wherein among two or more groups of dental pulp stem cells, a dental pulp stem cell in which the expression level of 10% or more of genes in the group of genes listed in Table 2 is 5 times or more as low as other groups of cells, is selected as the dental pulp stem cell to be used,
[14] The method according to any one of above [1] to [6] and [11] to [13], wherein among two or more groups of dental pulp stem cells, a dental pulp stem cell in which the expression level of at least one gene selected from the group consisting of Gafa3, Lmf1, Fam13a, Gkn1, Gpr112, Sult1c4, Slc35f4, Gstt1, Gpr27, Pdia2, RIIAD1, GSTT1, SLC2A2 and HTATSF1P2 is 5 times or more as low as other groups of cells, is selected as the dental pulp stem cell to be used,
[15] The method according to any one of above [1] to [14], wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes,
[16] A graft material for treating nerve damage, comprising a dental pulp stem cell, and a medium substantially containing no growth factors except FGF2,
[17] The graft material for treating nerve damage according to above [16], wherein the medium substantially containing no growth factors except FGF2 is a serum-containing base medium supplemented with FGF2 alone as a growth factor,
[18] The graft material for treating nerve damage according to above [17], wherein the medium substantially containing no growth factors except FGF2 is a commercially available medium for culturing mesenchymal stem cells supplemented with FGF2 alone as a growth factor,
[19] The graft material for treating nerve damage according to any one of above [16] to [18], wherein the dental pulp stem cell is a dental pulp stem cell in which the expression level of 10% or more of genes in the group of genes listed in Table 1 is 5 times or more as high as an average expression level of genes of dental pulp stem cells,
[20] The graft material for treating nerve damage according to any one of above [16] to [19], wherein the dental pulp stem cell used is a dental pulp stem cell in which the expression level of at least one gene selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as an average expression level of genes of dental pulp stem cells,
[21] The graft material for treating nerve damage according to any one of above [16] to [20], wherein the dental pulp stem cell used is a dental pulp stem cell in which the expression level of 10% or more of genes in the group of genes listed in Table 2 is 5 times or more as low as an average expression level of genes of dental pulp stem cells,
[22] The graft material for treating nerve damage according to any one of above [16] to [21], wherein the dental pulp stem cell used is a dental pulp stem cell in which the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as an average of dental pulp stem cells,
[23] The graft material for treating nerve damage according to any one of above [16] to [22], wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes,
[24] A method for treating nerve damage, comprising
   a step of grafting a graft material for treating nerve damage produced by the method according any one of above [1] to [15] or the graft material for treating nerve damage according to any one of [16] to [23] to an area of nerve damage,
[25] The method according to above [24], wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes,
[26] A kit for producing a graft material for treating nerve damage, comprising a medium and FGF2, and
[27] A method for selecting a material for a graft material for treating nerve damage from a plurality of groups of dental pulp stem cells, comprising selecting a dental pulp stem cell having at least one of the following properties (i) to (iv):
   (i) the expression level of 10% or more of genes in the group of genes listed in Table 1 is 5 times or more as high as other groups of cells,
   (ii) the expression level of at least one gene selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as other groups of cells,
   (iii) the expression level of 10% or more of genes in the group of genes listed in Table 2 is 5 times or more as low as other groups of cells, and
   (iv) the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as other groups of cells.

### Advantageous Effects of Invention

The graft material for treating nerve damage according to the present invention can be obtained in a simple method by adding FGF2 to a medium for culturing a dental pulp stem cell, and can provide a high motor function recovery effect by grafting the material.

The dental pulp stem cells, which is waste obtained from younger persons in a large amount and can be cryopreserved for a long term, are easily obtained. If the dental pulp stem cells derived from a person himself are used, a problem of immune rejection associated with grafting rarely occurs. Because dental pulp stem cells are tissue stem cells, growth of the cells is limited and thus a risk of cancerization is conceivably low compared to pluripotent stem cells.

### Brief Description of Drawings

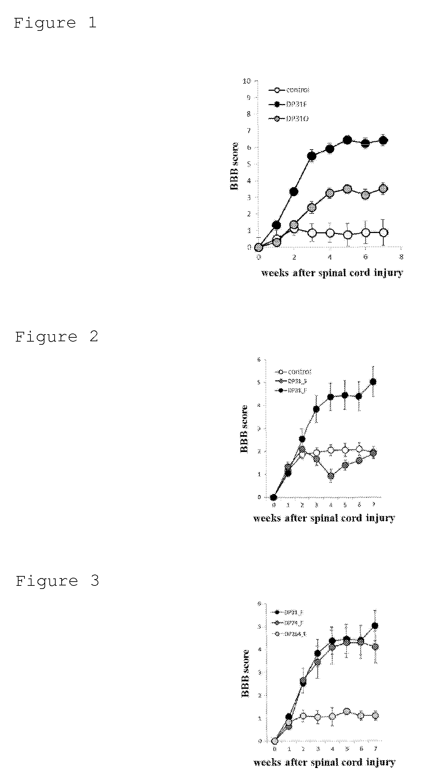

[Figure 1] Figure 1 is a graph showing the measurement results of motor function recovery based on BBB score when a graft material obtained by culture in a medium substantially containing no growth factors except FGF2 (DP31F), a graft material (DP310) produced by a conventional method, and a control graft material (control) were grafted to rat models with total amputation of spinal cord.
[Figure 2] Figure 2 is a graph showing the measurement results of motor function recovery based on BBB score when a graft material obtained by culture in a medium substantially containing no FGF2 (DP31S), a graft material obtained by culture in a medium substantially containing no growth factors except FGF2 (DP31F) and a control graft material (control) were grafted to rat models with total amputation of spinal cord [Figure 3] Figure 3 is a graph showing the measurement results of motor function recovery based on BBB score when graft materials obtained by culture in a medium substantially containing no growth factors except FGF2 (DP31F, DP74F, DP264F) to rat models with total amputation of spinal cord.

### Description of Embodiments

[Method for producing graft material for treating nerve damage]

An embodiment of the method for producing a graft material for treating nerve damage according to the present invention includes a step of culturing a dental pulp stem cell in a medium substantially containing no growth factors except FGF2.

In the specification, the "dental pulp stem cell" refers to a kind of tissue stem cell that can be isolated from the dental pulp. The tissue stem cell is also called as a somatic stem cell. Compared to an embryonic stem cell capable of differentiating into any types of cells, the tissue stem cell can be differentiated into limited types of cells.

The dental pulp stem cell can be collected from either one of a baby tooth and a permanent tooth and can be obtained from the dental pulp of an evulsion tooth such as a wisdom tooth and a baby tooth, which have been treated as medical waste. The dental pulp stem cell can be prepared and stored in accordance with methods known to those skilled in the art (for example, Takeda, T. et al.: J. Dent. Res., 87: 676-681, 2008; Tamaoki et al., J Dent Res. 2010 89: 773-778).

The dental pulp stem cell is a mesenchymal stem cell present in the hard tissue, similarly to the bone marrow mesenchymal stem cell, and can be subcultured in the same manner as in the bone marrow mesenchymal stem cell (for example, the method described in "Experimental Medicine, additional volume, Revised Cultured Cell Experiment Handbook, Chapter 8, Human Bone Marrow Mesenchymal Stem Cell", published January 1, 2009 (Yodosya)); however, the dental pulp stem cell has a long cellular division span and is not differentiated into a fat cell. Likewise, the dental pulp stem cell has different features from the stem cell isolated from the bone marrow.

The dental pulp stem cell is easily obtained and a culture method and a storage method for them are established, as described above. For the reasons, it has been expected to use the dental pulp stem cell as a base of a graft material for regenerative medicine. The dental pulp stem cells are collected from an evulsion tooth, after that, if necessary, proliferated by culturing to a predetermined amount. Since dental pulp stem cells can be cryopreserved for a long time, if dental pulp stem cells are isolated from many people and stored, a dental pulp stem cell bank can be formed.

The dental pulp stem cell is characterized by, for example, surface antigen STRO-1. Other than this, the dental pulp stem cell can be distinguished by a neural crest cell marker such as Nestin, SOX10 and SOX11 used as an index.

The dental pulp stem cell is known to have a high proliferation potency, compared to a bone marrow mesenchymal stem cell. It is also known that if the dental pulp stem cell is grafted together with calcium phosphate or hydroxyapatite to a mouse, dentin is formed.

In the method for producing a graft material for treating nerve damage according to the present invention, cells derived from a recipient of grafting or cells derived from a person except the recipient may be used. For example, dental pulp stem cells are isolated from e.g., a baby tooth or a wisdom tooth of a recipient of grafting, cultured and cryopreserved, and then, thawed at the time of need and used. Alternatively, a dental pulp stem cell having an identical human leukocyte antigen (HLA) with that of a recipient of grafting may be selected from the dental pulp stem cell bank and used for producing a graft material for treating nerve damage.

In the specification, the "nerve damage" refers to a damage in the central nerve and the peripheral nerve. Examples thereof include, but are not limited to, spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes. The graft material of the present invention can be applied to any nerve damages as long as a therapeutic effect can be obtained by grafting. The therapeutic effect refers to an effect of curing a disease; however, the therapeutic effect is not limited to this and includes an effect of improving at least one symptom associated with a disease and an effect of inhibiting or delaying progression of a disease,and others.

In the specification, the graft recipient is not limited to humans and may include other mammals (for example, mice, rats, rabbits, dogs, cats, monkeys, sheep, cows, horses).

In the method for producing a graft material for treating nerve damage according to the present invention, a dental pulp stem cell is cultured in a medium substantially containing no growth factors except FGF2.

In the specification, the "medium substantially containing no growth factors except FGF2" means that the growth factor to be purposely added is FGF2 alone. Examples of such a medium include a serum-containing base medium supplemented with FGF2 alone as a growth factor; a serum-free base medium supplemented with FGF2 alone as a growth factor; a serum-containing base medium supplemented with FGF2 alone as a growth factor; a medium commercially available for culturing mesenchymal stem cells supplemented with FGF2 alone as a growth factor; and a medium commercially available for culturing mesenchymal stem cells supplemented with FGF2 alone as a growth factor.

In the specification, the "base medium" refers to a medium containing known low molecular-weight components alone. Non-limiting examples of the base medium known in the art include Eagle mediums such as BME (Basal medium Eagle's), MEM (Minimum essential medium) and DMEM (Dulbecco's modified Eagle's medium); RPMI (Roswell Park Memorial Institute) mediums such as RPMI1630 and RPMI1640; Fischer's medium, Ham's mediums such as F10 medium and F12 medium, MCDB mediums such as MCDB104, 107, 131, 151, 153, 170 and 202; and RITC80-7 medium. The base medium can be appropriately selected from these.

In the specification, "the serum" refers to the supernatant obtained by clotting blood, more specifically, refers to blood from which cell components and coagulation proteins are removed. The serum to be used in the present invention may be derived from any animal. Examples thereof include human serum, fetal calf serum and horse serum. When a graft material for treatment according to the present invention is grafted to a human, human serum is preferable.

Since the serum contains various growth factors, the "serum-containing base medium" basically contains such growth factors. However, a medium containing growth factors except FGF2 at the levels equivalent to those of the serum is defined to be the "medium substantially containing no growth factors except FGF2" in the specification. The concentration of the serum in the medium is preferably e.g., less than 15 wt%, less than 13 wt%, less than 10 wt%, less than 8 wt% or less than 5 wt%.

In the specification, the "growth factor" refers to any type of protein called a growth factor or a proliferative factor. Examples thereof include epidermal growth factor (EGF), fibroblast growth factor (FGF), acid fibroblast growth factor (aFGF or FGF1), basic fibroblast growth factor (bFGF or FGF2), platelet-derived growth factor (PDGF), nerve growth factor (NGF), insulin-like growth factor (IGF), hepatocyte growth factor (HGF), transforming growth factor (TGF), vascular endothelial growth factor (VEGF) and keratinocyte growth factor (KGF) interleukins, and others.

In the specification, the "medium commercially available for culturing mesenchymal stem cells" refers to a commercially available medium for culturing and proliferating mesenchymal stem cells while maintaining a differentiation potency and not inducing differentiation. Examples thereof include, but are not limited to, MSCGM medium (LONZA), mesenchymal stem cell growth medium (Takara Bio Inc.), mesenchymal stem cell growth medium DXF (Takara Bio Inc.), StemLine (registered trade mark) mesenchymal stem cell growth medium (Sigma-Aldrich), MF-medium (trade mark) mesenchymal stem cell growth medium (Toyobo Life Science), BD Mosaic (trade mark) and a serum-free culture kit for human mesenchymal stem cells (BD BIOSCIENCES). Since some of these commercially available mediums contain secret components and low-level serum, mediums occasionally contain various growth factors; however, as long as the growth factor to be purposely added to these mediums is FGF2 alone, these mediums correspond to the "medium substantially containing no growth factors except FGF2" of the present invention. In this case, the level of the serum is preferably less than 15%, less than 13%, less than 10%, less than 8% or less than 5%.

In the specification, FGF2 refers to a basic fibroblast growth factor (FGF) and also referred to as bFGF or HBGF-2.

FGF2 used herein can be prepared by appropriately diluting a commercially available FGF2. Since FGF2 is to be used in a graft material, FGF2 is filtered by an appropriate membrane and preferably confirmed to be negative to e.g., bacteria, fungi and mycoplasma. The concentration of FGF2 is not particularly limited as long as the resultant graft material has a sufficient spinal cord injury therapeutic effect; however, the concentration can be specified as, for example, 5 ng/mL or more or 7 ng/mL or more.

In the specification, the "medium supplemented with FGF2 alone as a growth factor" may contain e.g., other proteins as long as a growth factor except FGF2 is not added.

Examples of the substance to be added to the medium include hormones such as insulin, glucagon, prolactin, thyroxine, growth hormone, follicle stimulating hormone (FSH), luteinizing hormone (LH), thyroid hormone, estradiol and glucocorticoid; binding proteins such as ceruloplasmin, transferrin and lipoprotein; cell adhesion factors such as collagen, fibronectin, laminin and vitronectin; lipids such as prostaglandins, phospholipids and unsaturated fatty acids; and various low molecular-weight compounds. These can be used singly or in arbitrary combination. The concentrations of these substances may be appropriately selected by those skilled in the art.

To the medium to be used in the method for producing a graft material for treating spinal cord injury according to the present invention, other substances useful for culturing cells can be appropriately added. Examples of the substances include, but are not limited to, a buffer for stabilizing pH (e.g., HEPES), phenol red serving as a pH indicator, antibiotic substances (e.g., penicillin G, streptomycin, amphotericin B, gentamicin, kanamycin, ampicillin, minocycline, gentashin), amino acids, vitamins, lipids, carbohydrates, nucleic acids, inorganic salts, organic acid salts and minerals, and others.

The medium to be used in the method for producing a graft material for treating spinal cord injury according to the present invention can be prepared by dissolving requisite components in water, a buffer or a commercially available medium.

As the water to be used for preparing the medium, ultra-pure water compatible to pure water for injection is desirably used.

The medium is also aseptically prepared in a high-standard clean room or a clean bench and dispensed through a sterile filter having a pore size of 0.1 µm or less and capable of removing mycoplasma.

The storage container of the medium is preferably a plastic container made of e.g., a poly (ethylene terephthalate) co-polymer rather than a glass container, since proteins are likely to adsorb to the inner wall of the glass container.

The medium prepared may be subjected to various quality evaluation tests (such as physical property tests including measurement of e.g., pH and osmotic pressure; microorganism tests for examining contamination with e.g., bacteria, fungi and mycoplasma; virus tests for examining contamination with e.g., hepatitis virus and HIV; measurement for endotoxin level; and tests for biological activities such as cell proliferation and physiological function).

In the method for producing a graft material for treating nerve damage according to the present invention, it is preferable that a dental pulp stem cell is also subcultured in the aforementioned medium, twice, 3 times, 4 times, 5 times or 6 times or more.

The culture method is not particularly limited as long as culture is carried out in a medium substantially containing no growth factors except FGF2. Various conditions (such as temperature, humidity, CO₂ concentration, pH, frequency of exchanging medium) can be selected by those skilled in the art depending on the type of cell to be cultured.

The culture period can be appropriately determined by those skilled in the art depending upon the type of cell and the composition of the medium. For example, whether cells reach the state suitable for grafting may be determined based on the shape of the cells and the proliferation rate thereof by those skilled in the art. As the state suitable for grafting, for example, the state where the shape of a cell changed into a thin and long shape and the state where cell proliferation speed decreases may be mentioned, but not limited to these states.

In the method for producing a graft material for treating nerve damage according to the present invention, cells may be cultured by any method such as a single layer stationary culture, a rotary culture, a microcarrier culture, a suspension culture, a gyratory culture, a spheroid culture, a culture within gel and a culture by a three-dimensional carrier.

The single layer stationary culture is a method of culturing cells of a single layer by attaching the cells on the wall of a culture container. A glass or plastic culture container may be used. As the plastic, a plastic whose surface has been treated to be appropriately hydrophilic, can be used. Depending upon the type of cell and the purpose of an experiment, the plastic may be coated with an extracellular matrix such as collagen, gelatin, laminin, fibronectin and matrigel. As the coating material, collagen crosslinked by UV irradiation and gelatin obtained by treating collagen with heat can be used.

The rotatory culture is a culture method by placing a culture container in a rotatory metal drum. Large scale culture can be made if e.g., a bottle type culture container is used.

The microcarrier culture is a culture method using carriers like beads. More specifically, cells are allowed to adhere to the surface of the beads and culture is made by stirring a medium containing the beads and suspending them. This method is suitable for large-scale culture.

The suspension culture is a method of culturing cells while suspending the cells in a medium. Adhesive cells may be forcibly suspended by stirring the medium and cultured. A large amount of cells can be collected compared to the single layer culture.

The gyratory culture refers to a culture method by horizontally rotating a culture container. This is used as one of the suspension cultures and also used for forming spheroids taking advantage of a nature: suspended solids assemble to the center by gyration.

The spheroid culture is a method for forming spheroids through mutual adhesion of cells by suspending cells such that the cells are in loose contact with each other. Many of the cells obtained by the spheroid culture highly express function.

The culture within gel is a method of culturing cells by embedding the cells within e.g., collagen gel, soft agar or synthetic polymer gel and suitable for three-dimensional culture.

The three-dimensional carrier culture is a culture method using a carrier so as to three-dimensionally proliferating cells at a high density in order to enhance expression of function of cultured cells. As the carrier, a porous polymer and beads are generally used. To facilitate nutrition and gas exchange of cells densely present, a circulation system by a bioreactor is employed.

The method for producing a graft material for treating nerve damage according to the present invention may include, in addition to the aforementioned culture step, various steps appropriate for producing a graft material. For example, a step of controlling flowability of the culture obtained in the culture step by mixing the culture with a highly viscose substance such as hyaluronic acid, collagen gel, fibrinogen, soft agar and a synthetic polymer, may be carried out. By appropriately controlling flowability, a graft material can be settled at a damage site.

After mixing with gel such as collagen gel, soft agar or a synthetic polymer, culture is performed in a certain period of time and then a three-dimensional culture may be performed.

The dental pulp stem cell to be used for producing a graft material for treating nerve damage according to the specification may be a dental pulp stem cell in which the expression level of the group of genes listed in Table 1 is high compared to the average expression level of genes in dental pulp stem cells.

In the specification, the "dental pulp stem cell, in which the expression level of the group of genes listed in Table 1 is high compared to the average expression level of genes in dental pulp stem cells" refers to a dental pulp stem cell satisfying the following condition: when gene expression pattern was checked with respect to expression of the group of genes described in Table 1, the expression levels of 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 98% or more of genes, each are 5 times or more, 10 times or more, 20 times or more, 30 times or more, 40 times or more, 50 times or more, 60 times or more, 70 times or more, 80 times or more, 90 times or more, 95 times or more, or 100 times or more as high as the average in dental pulp stem cells.

In the specification, the "average expression level of genes in dental pulp stem cells" refers to an average of expression levels of genes in an arbitrary number (two or more) of dental pulp stem cells.

[Table 1]

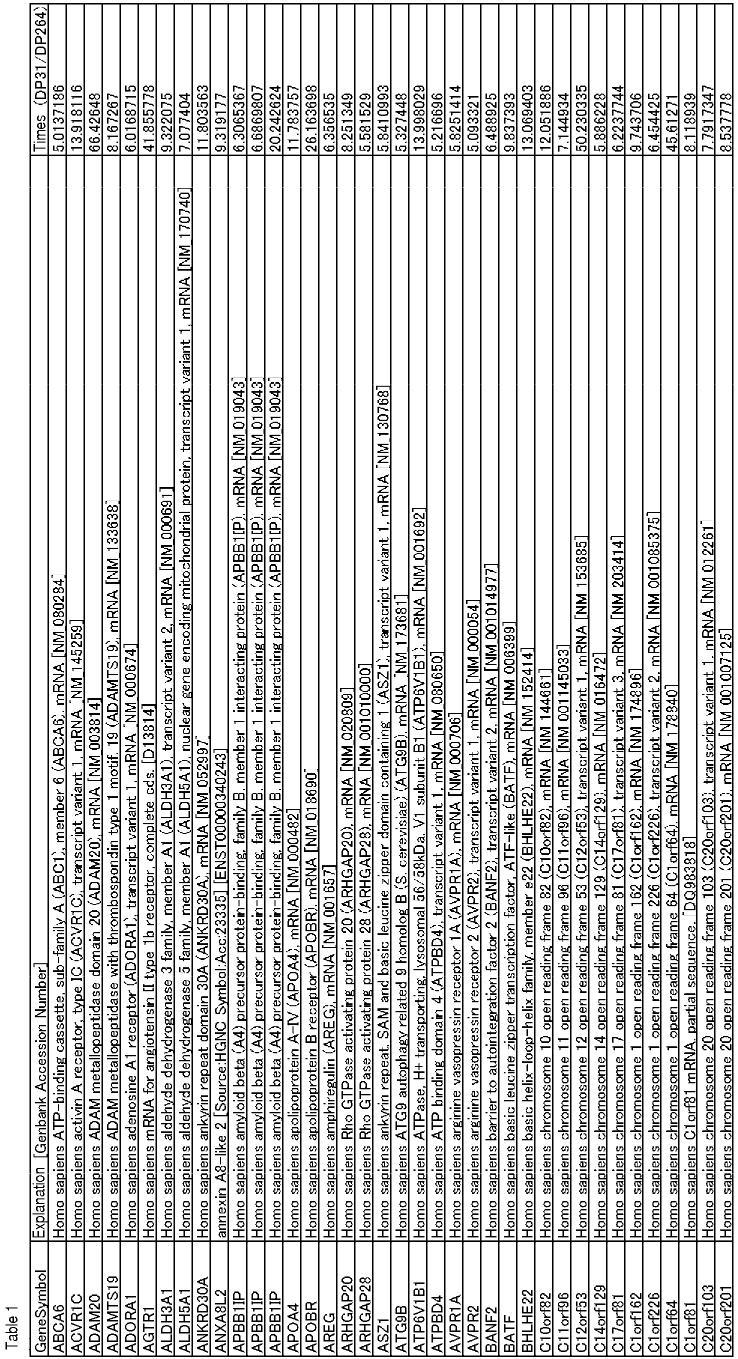

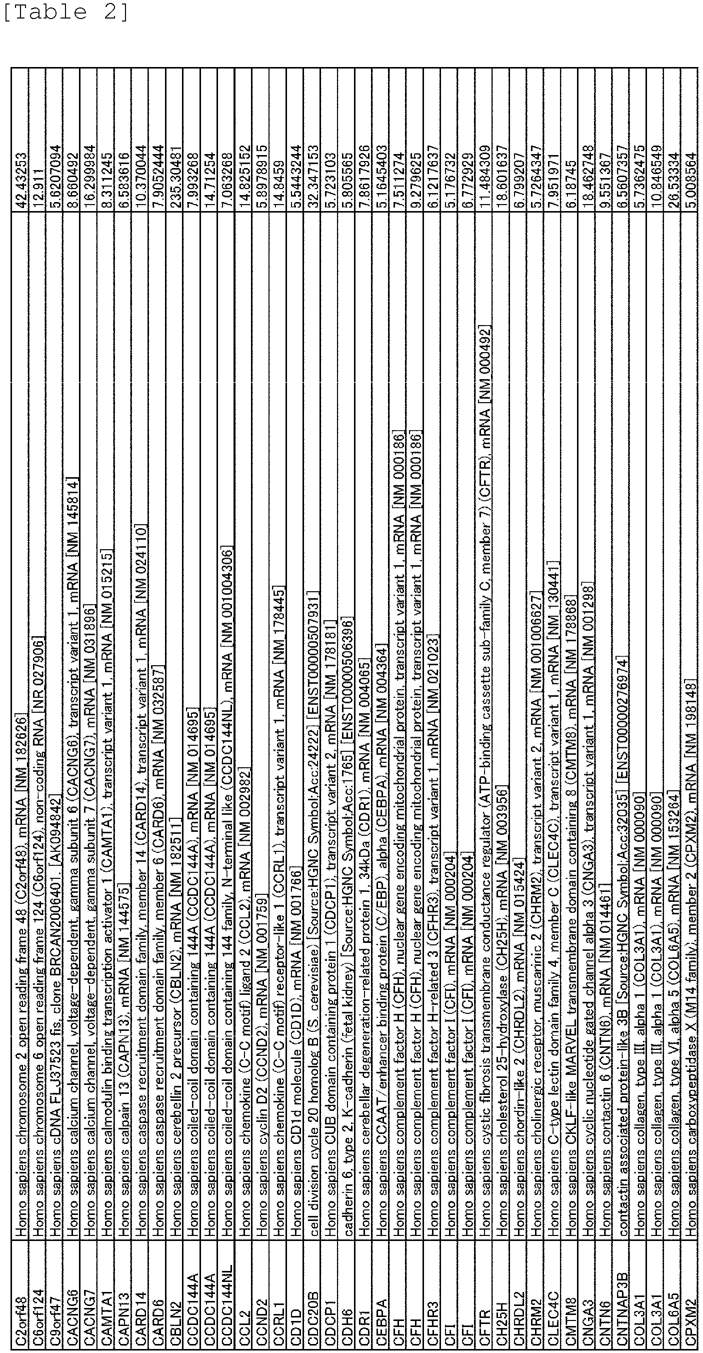

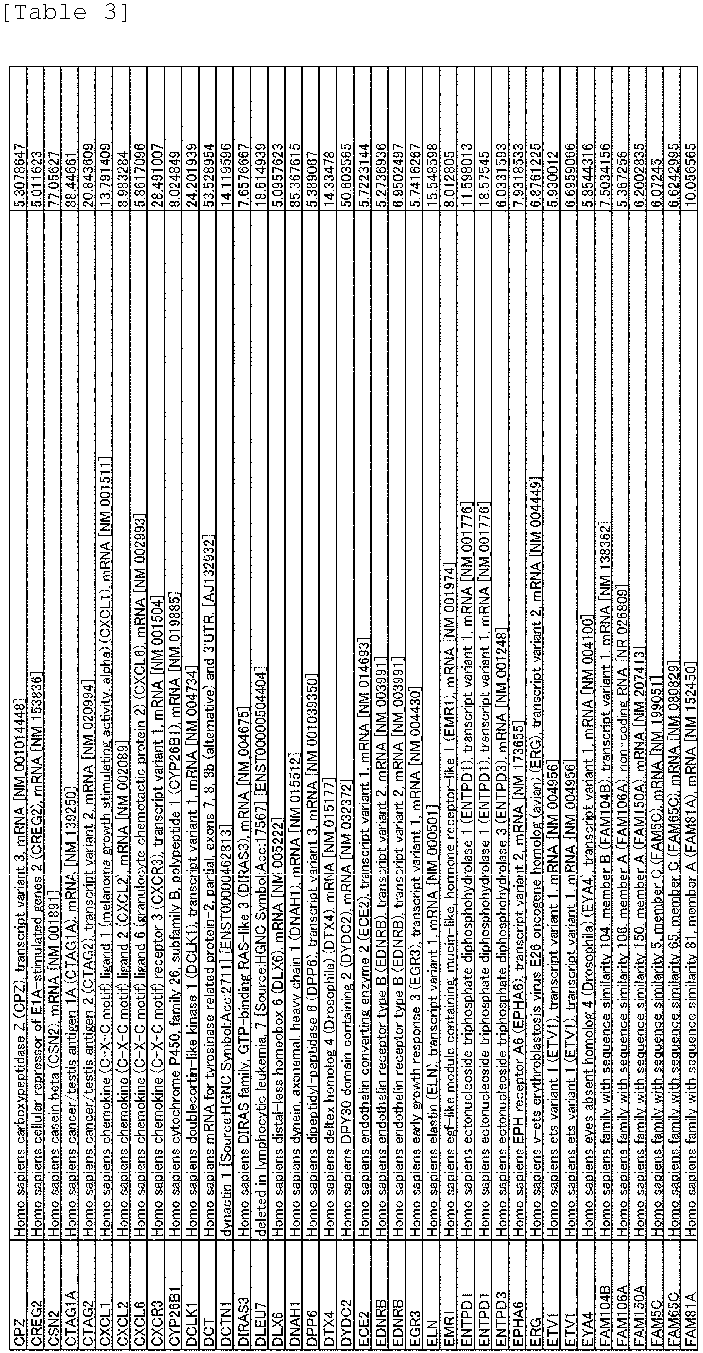

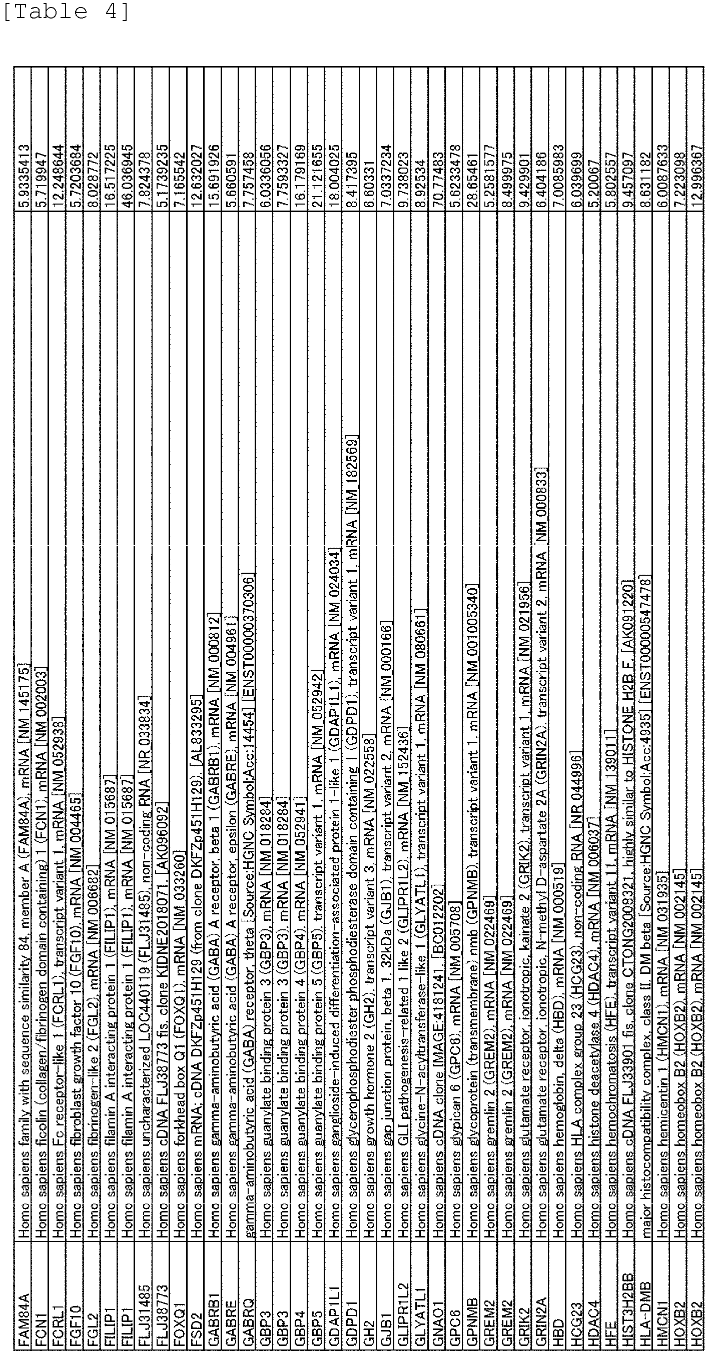

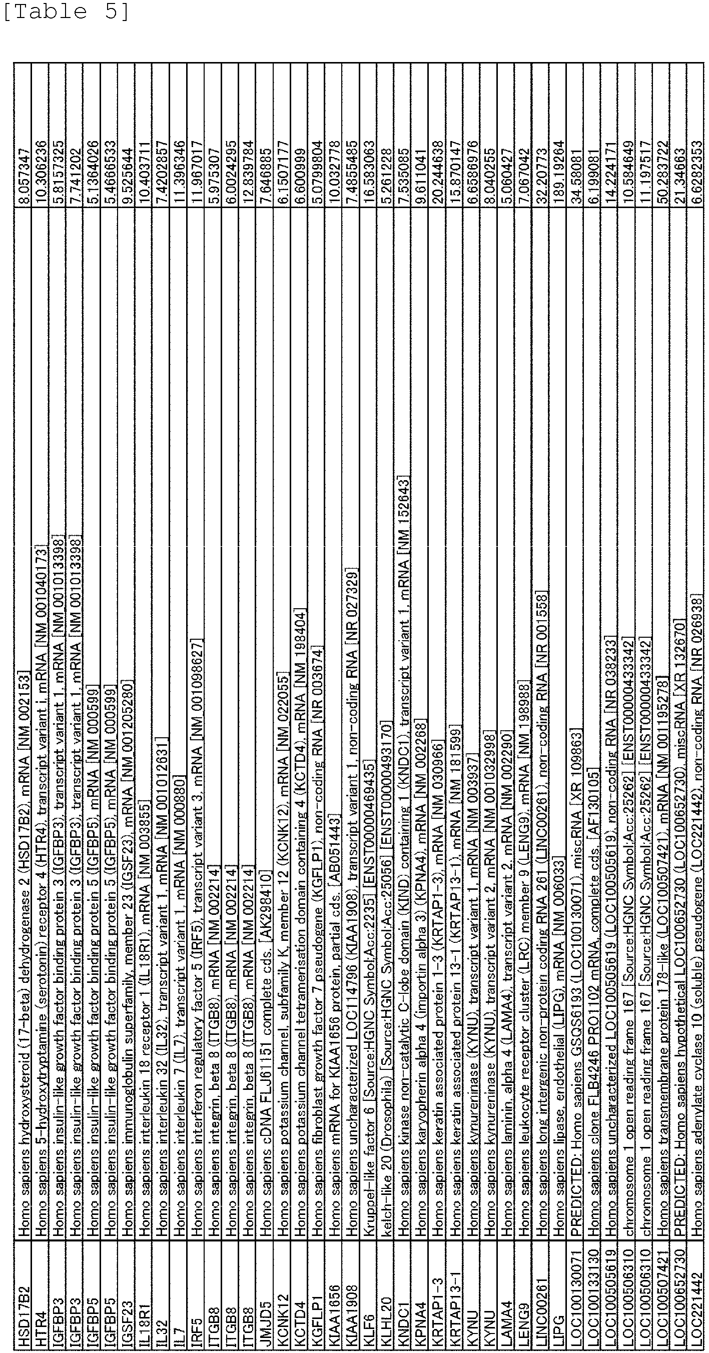

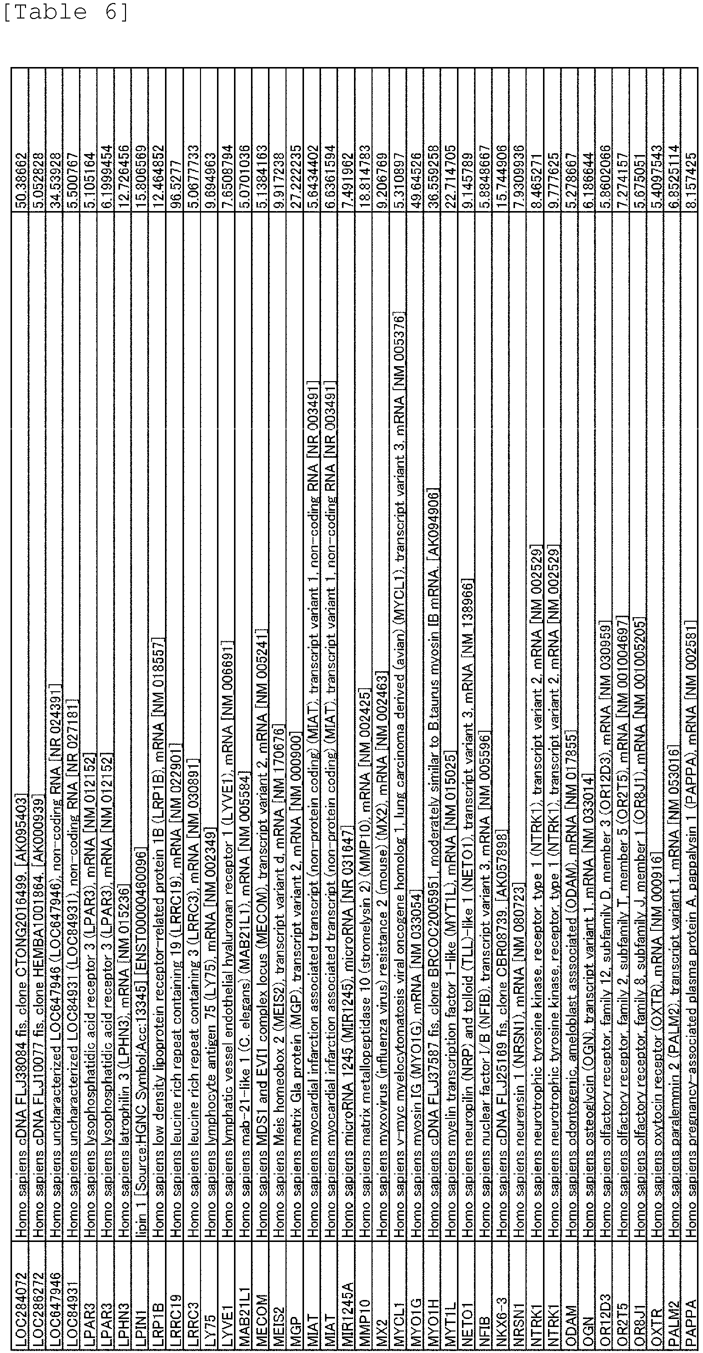

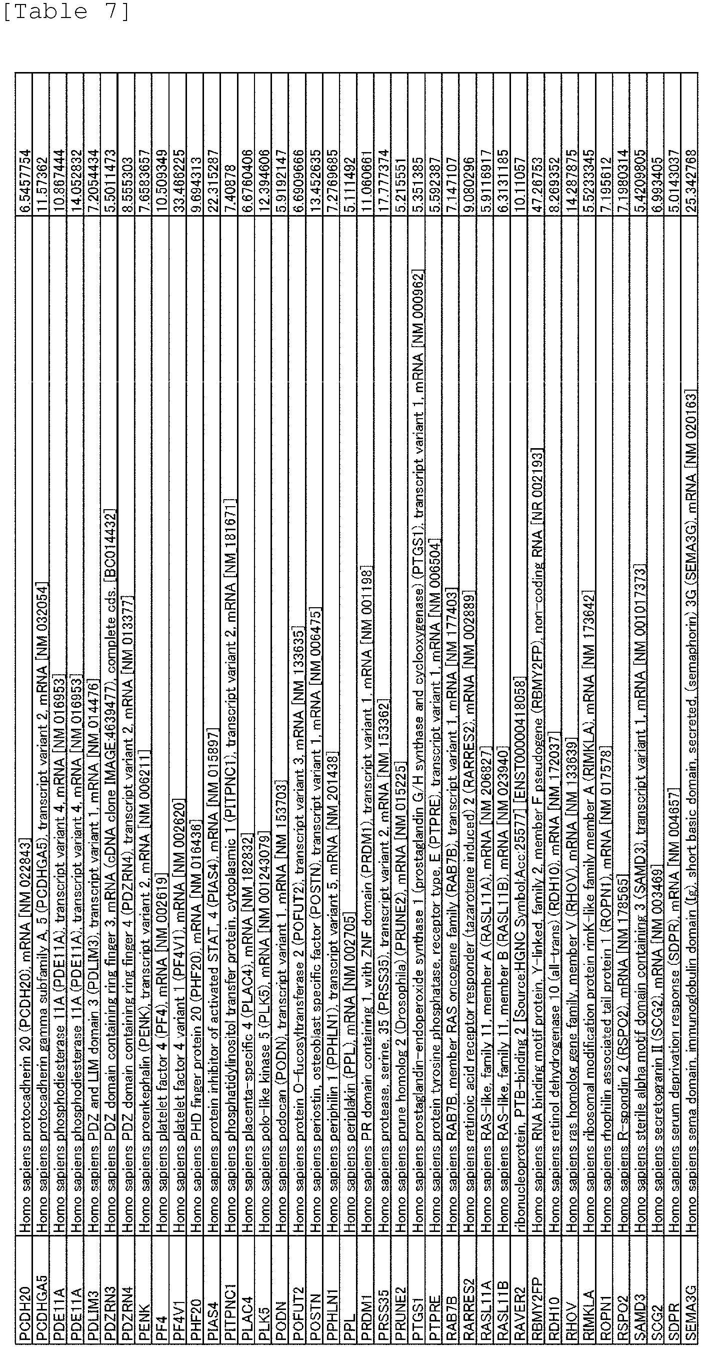

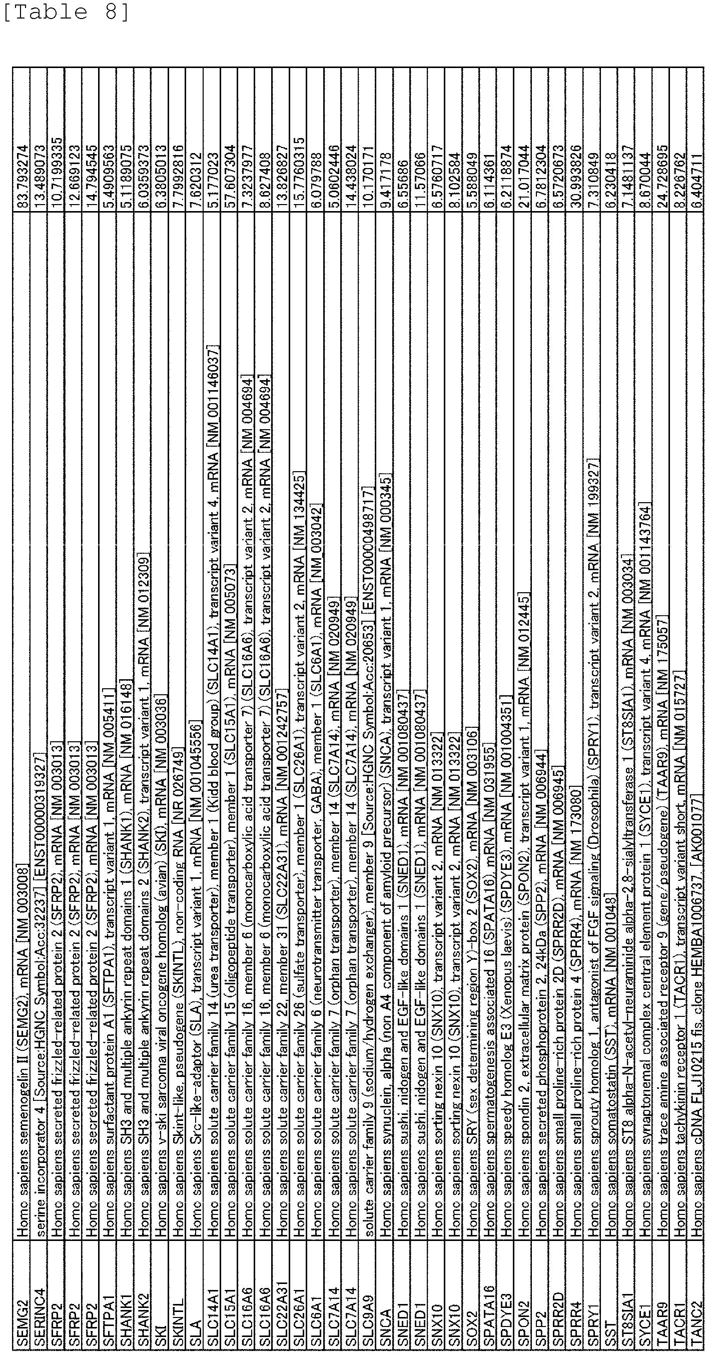

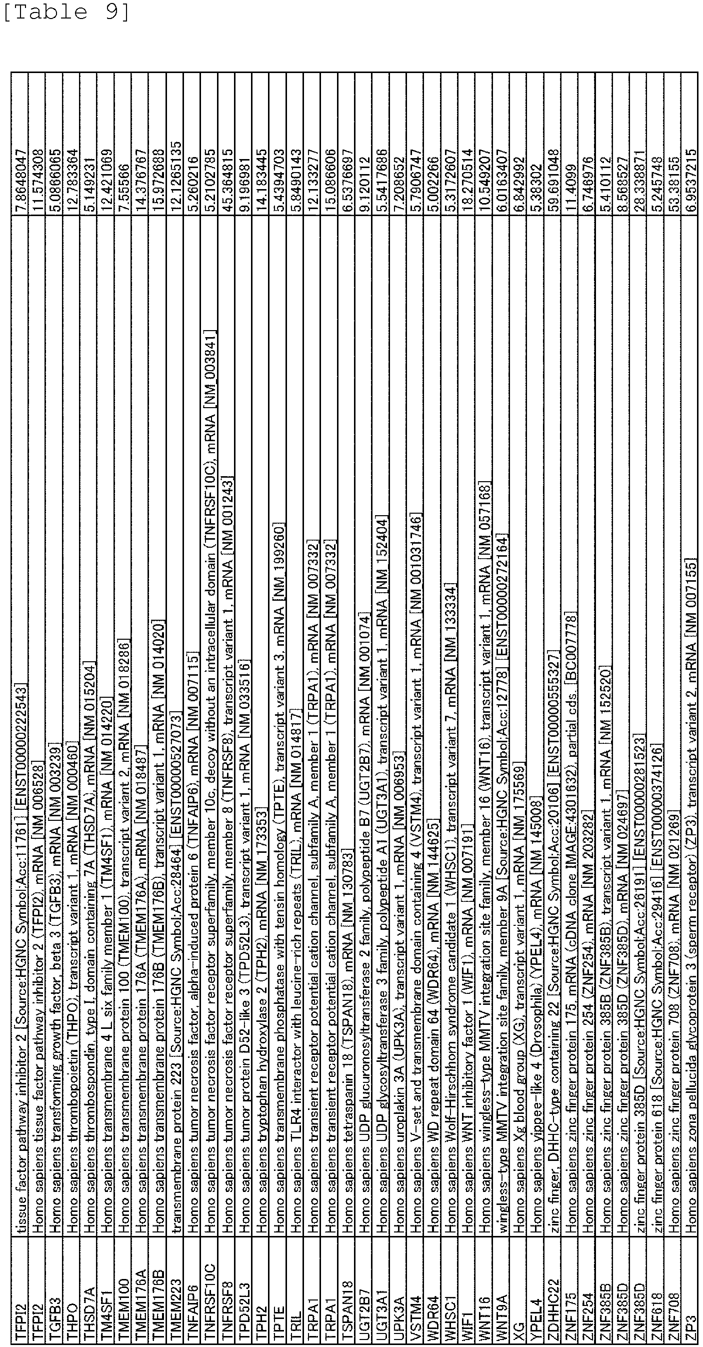

The dental pulp stem cell to be used in producing a graft material for treating nerve damage according to the specification may be a dental pulp stem cell in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more, 14 types or more, 15 types or more, 16 types or more, 17 types or more, 18 types or more, 19 types or more, 20 types or more, or 21 types of genes selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg, and Cbln2 are each 5 times or more, 10 times or more, 20 times or more, 30 times or more, 40 times or more, 50 times or more, 60 times or more, 70 times or more, 80 times or more, 90 times or more, 95 times or more, or 100 times or more, each are as high as an average expression level thereof in the dental pulp stem cells.

Among two or more groups of dental pulp stem cells, a group of dental pulp stem cells in which the gene expression level of the group of genes listed in Table 1 is high may be selected and used as the dental pulp stem cell to be used in producing a graft material for treating nerve damage according to the specification.

More specifically, a group of dental pulp stem cells in which the expression levels of the genes of 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 98% or more of the group of genes described in Table 1, each are 5 times or more, 10 times or more, 20 times or more, 30 times or more, 40 times or more, 50 times or more, 60 times or more, 70 times or more, 80 times or more, 90 times or more, 95 times or more, or 100 times or more as high as other groups of cells, may be selected and used.

Among two types or more groups of dental pulp stem cells, a group of dental pulp stem cells in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more, 14 types or more, 15 types or more, 16 types or more, 17 types or more, 18 types or more, 19 types or more, 20 types or more, or 21 types of genes selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg, and Cbln2, each are 5 times or more, 10 times or more, 20 times or more, 30 times or more, 40 times or more, 50 times or more, 60 times or more, 70 times or more, 80 times or more, 90 times or more, 95 times or more, or 100 times or more as high as those of other groups of cells, may be selected and used as the dental pulp stem cell to be used in producing a graft material for treating nerve damage according to the specification.

In the specification, the "two types or more groups of dental pulp stem cells" refer to, for example, groups of dental pulp stem cells derived from two or more individuals; groups of the cells collected from a single individual at the intervals of a predetermined time; and groups of the cells derived from different teeth of a single individual. A group of cells may be a cell line proliferated from a single cell or a group of cells obtained by culturing a plurality of cells derived from a single individual.

In the specification, the type and level of gene expression can be examined by techniques known to those skilled in the art including Northern blotting, *in-situ* hybridization, RNAse protection assay and reverse transcription polymerase chain reaction (RT-PCR); however the techniques are not limited to these.

The dental pulp stem cell to be used in producing a graft material for treating nerve damage according to the specification may be a dental pulp stem cell in which the gene expression level of the group of genes listed in Table 2 is low compared to an average expression level in dental pulp stem cells.

In the specification, the "dental pulp stem cell, in which the gene expression level of the group of genes listed in Table 2 is low compared to an average expression level in dental pulp stem cells" refers to a dental pulp stem cell satisfying the following condition: when gene expression pattern was checked with respect to expression of the group of genes described in Table 2, the expression levels of genes corresponding to 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 98% or more of the group of genes described in Table 2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as the average expression level in dental pulp stem cells.

[Table 10]

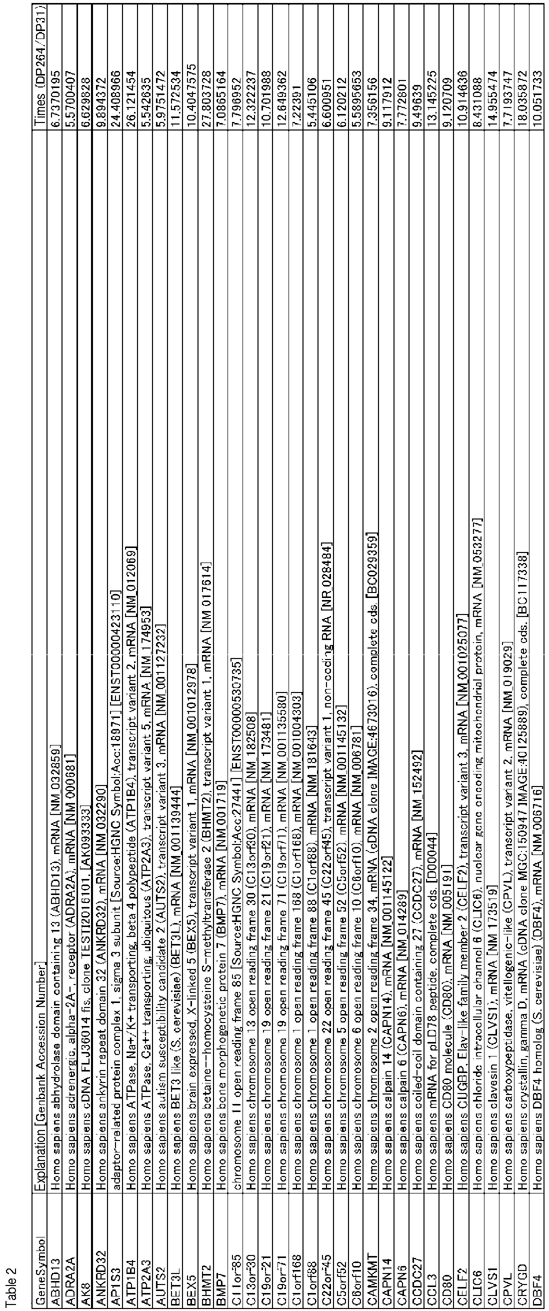

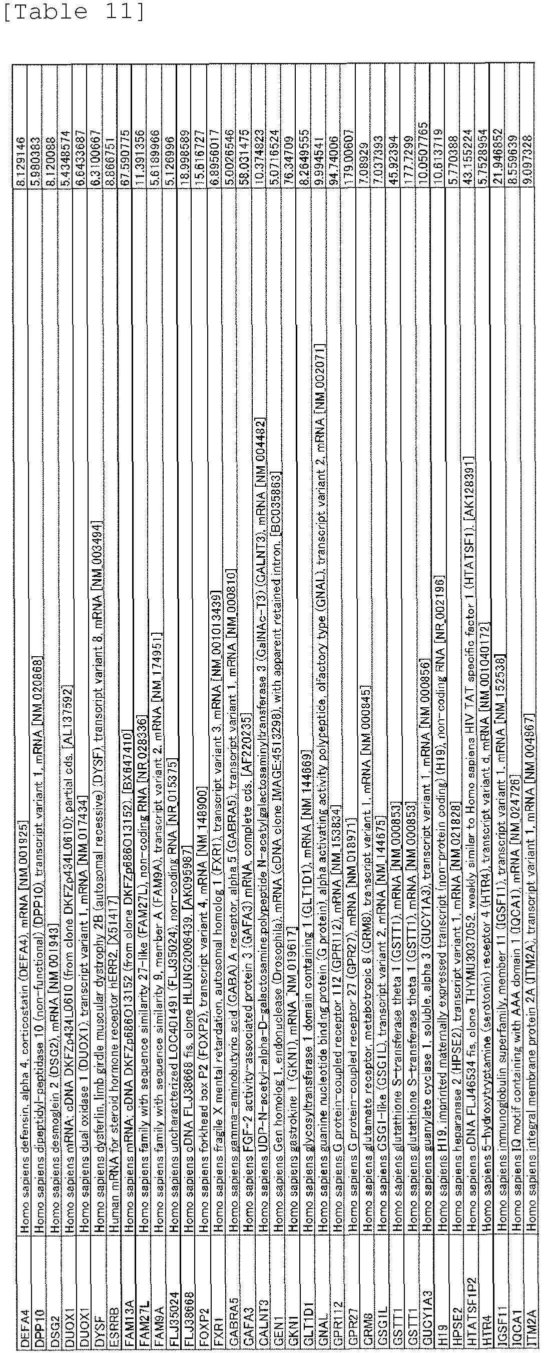

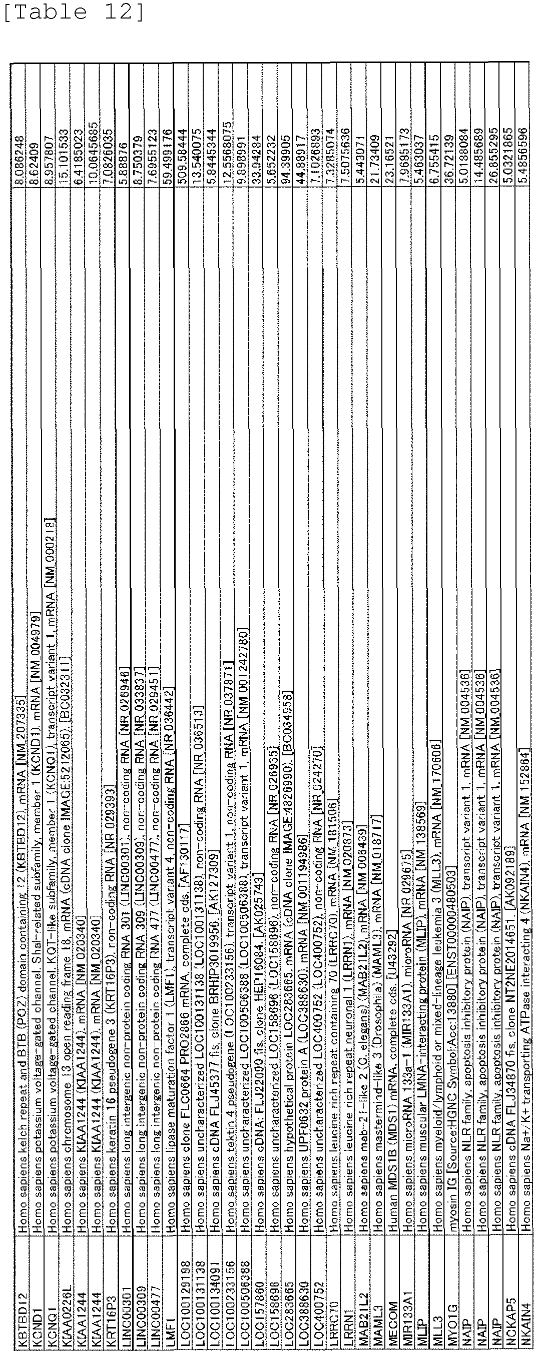

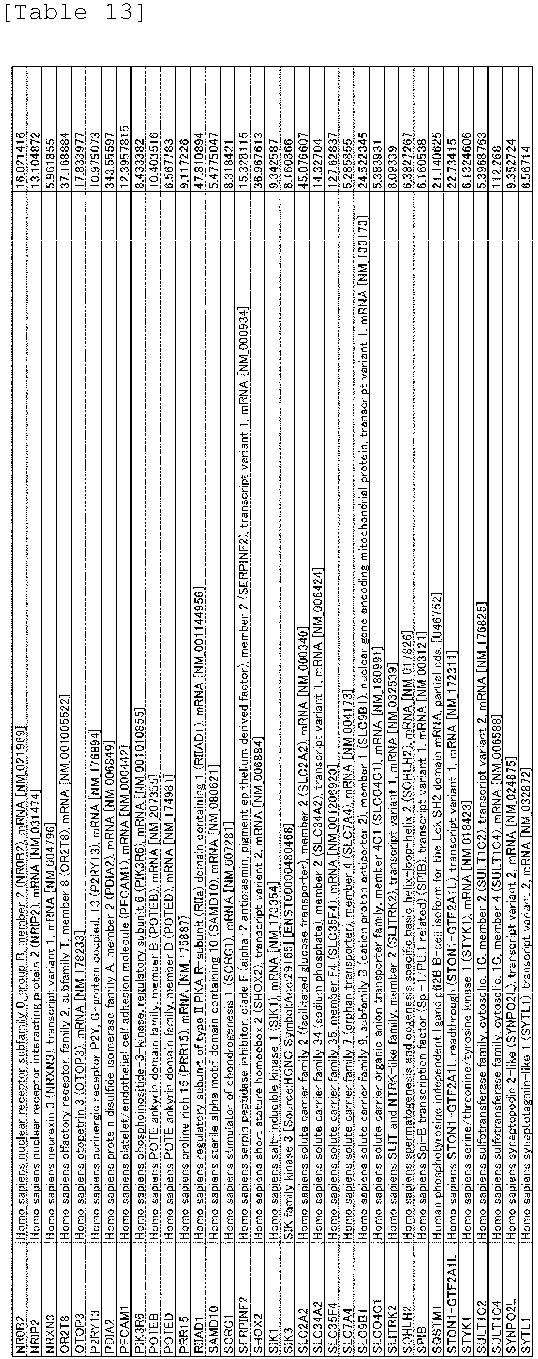

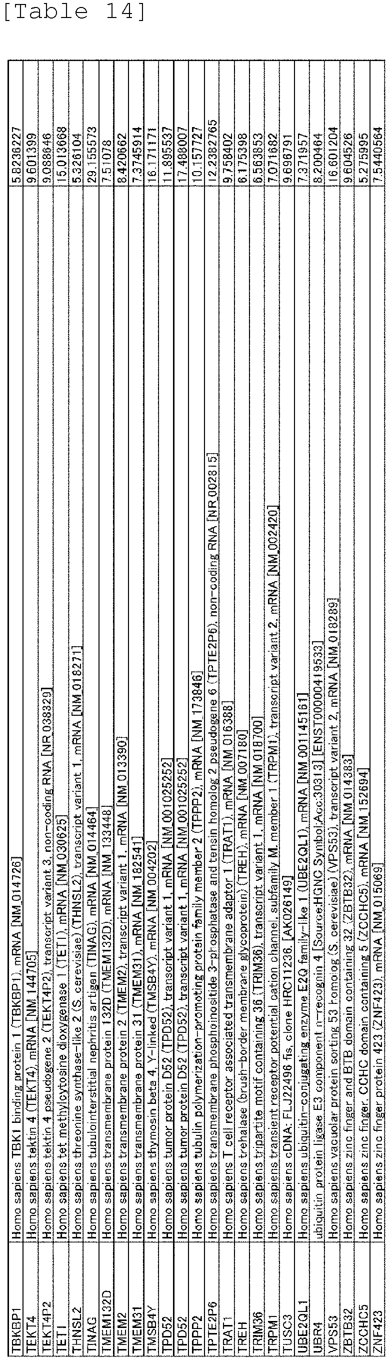

The dental pulp stem cell to be used in producing a graft material for treating nerve damage according to the specification may be a dental pulp stem cell in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more or 14 types of genes selected from the group consisting of Gafa3, Lmf1, Fam13a, Gkn1, Gpr112, Sult1c4, Slc35f4, Gstt1, Gpr27, Pdia2, RIIAD1, GSTT1, SLC2A2, and HTATSF1P2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as an average expression level in the dental pulp stem cells.

Among two types or more groups of dental pulp stem cells, a group of dental pulp stem cells in which the gene expression level of the group of genes listed in Table 2 is low, may be selected and used as the dental pulp stem cell to be used in producing a graft material for treating nerve damage according to the specification.

More specifically, a group of dental pulp stem cells in which the expression levels of the genes of 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 98% or more of the group of genes described in Table 2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as other groups of cells, may be selected and used.

Among two types or more groups of dental pulp stem cells, a group of dental pulp stem cells in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more or 14 types of genes selected from the group consisting of Gafa3, Lmf1, Fam13a, Gkn1, Gpr112, Sult1c4, Slc35f4, Gstt1, Gpr27, Pdia2, RIIAD1, GSTT1, SLC2A2 and HTATSF1P2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as other groups of cells, may be selected and used as the dental pulp stem cell to be used in producing a graft material for treating nerve damage according to the specification.

The effect of the graft material for treatment obtained by the method for producing a graft material for treating nerve damage according to the present invention can be evaluated by the following method using, for example, a disease-model animal.

A rat middle cerebral artery occlusion (MCAO) model can be prepared by a known method and more specifically prepared as follows. A wild type rat was anesthetized. While maintaining the rectal temperature at 37 ± 0.5°C under anesthesia, the cervical region was dissected to expose a branched part of the right carotid artery and internal and external carotid arteries were separated. Thereafter, a 4-0 nylon thin thread having a tip rounded off by silicon coat was inserted from the external carotid artery, allowed to reach the beginning of the middle cerebral artery through the internal carotid artery and fixed there. In this manner, blood flow in the right side middle cerebral artery region was blocked to cause ischemia. After the ischemia state was maintained for one hour, the nylon thin thread was withdrawn out of the middle cerebral artery to allow perfusion to start again.

At the 48th hour after the perfusion was started again, an effective amount of graft material (dental pulp stem cell) for treatment according to the present invention was administered from the caudal vein. Alternatively, the effective amount of graft material (dental pulp stem cell) for treatment may be locally administered to an infarction site.

Recovery of motor function is evaluated based on BBB score (Basso DM et al., J Neurotrauma. 1995 Feb; 12 (1): 1-21).

### [Graft material for treating nerve damage]

The graft material for treating nerve damage according to the present invention is produced by the method for producing a graft material for treating nerve damage according to the present invention described above and contains a dental pulp stem cell and a medium substantially containing no growth factors except FGF2. The "dental pulp stem cell" and the "medium substantially containing no growth factors except FGF2" are the same as defined above. The graft material may contain gel such as collagen gel, soft agar and a synthetic polymer and the viscosity may be controlled by an appropriate gelation agent or a thickening agent.

The dental pulp stem cell to be used in a graft material for treating nerve damage according to the specification may be a dental pulp stem cell in which the gene expression level of the group of genes listed in Table 1 is high compared to the average expression level of genes in dental pulp stem cells. The "dental pulp stem cell, in which the gene expression level of the group of genes listed in Table 1 is high compared to the average expression level of genes in dental pulp stem cells" is the same as described above.

The graft material for treating nerve damage according to the specification may be a graft material using a dental pulp stem cell in which compared to an average expression level in the dental pulp stem cells, the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more, 14 types or more, 15 types or more, 16 types or more, 17 types or more, 18 types or more, 19 types or more, 20 types or more, or 21 types of genes selected from the group consisting of MYO1G, RBMY2FP, FILIP1, C1orf64, TNFRSF8,C2orf48, AGTR1, Dydc2, Znf708, Dct, Slc15a1, Zhddc22, Adam20, Gnao1, Csn2, Semg2, Dnah1, Ctag1a, Lrrc19, Lipg, and Cbln2, each are 5 times or more, 10 times or more, 20 times or more, 30 times or more, 40 times or more, 50 times or more, 60 times or more, 70 times or more, 80 times or more, 90 times or more, 95 times or more, or 100 times or more as high as other groups of cells.

As the dental pulp stem cell to be used in the graft material for treating nerve damage according to the specification, a group of dental pulp stem cells in which the gene expression level of the group of genes listed in Table 1 is high may be selected and used from two types or more groups of dental pulp stem cells and used. The "dental pulp stem cells in which the gene expression level of the group of genes listed in Table 1 is high, may be selected and used from two types or more groups of dental pulp stem cells" is the same as defined above.

Among two types or more groups of dental pulp stem cells, a group of dental pulp stem cells in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more, 14 types or more, 15 types or more, 16 types or more, 17 types or more, 18 types or more, 19 types or more, 20 types or more, or 21 types of genes selected from the group consisting of MYO1G, RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg, and Cbln2, each are 5 times or more, 10 times or more, 20 times or more, 30 times or more, 40 times or more, 50 times or more, 60 times or more, 70 times or more, 80 times or more, 90 times or more, 95 times or more, or 100 times or more as high as other groups of cells, may be selected and used as the dental pulp stem cell to be used in the graft material for treating nerve damage according to the specification.

The dental pulp stem cell to be used in the graft material for treating nerve damage according to the specification may be a dental pulp stem cell, in which the gene expression level of the group of genes listed in Table 2 is low compared to an average expression level in dental pulp stem cells.

The "dental pulp stem cell, in which the gene expression level of the group of genes listed in Table 2 is low compared to an average expression level in dental pulp stem cells" is the same as described above.

The dental pulp stem cell to be used in the graft material for treating nerve damage according to the specification may be a dental pulp stem cell, in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more or 14 types of genes selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as an average expression level in the dental pulp stem cells.

The dental pulp step cell to be used in the graft material for treating nerve damage according to the specification includes a graft material using a dental pulp stem cell in which the gene expression level of the group of genes listed in Table 2 is low, of two types or more groups of dental pulp stem cells.

The "dental pulp stem cell, in which the gene expression level of the group of genes listed in Table 2 is low, of two types or more groups of dental pulp stem cells" is the same as described above.

Among two types or more groups of dental pulp stem cells, a group of dental pulp stem cells, in which the expression levels of one type or more, two types or more, 3 types or more, 4 types or more, 5 types or more, 6 types or more, 7 types or more, 8 types or more, 9 types or more, 10 types or more, 11 types or more, 12 types or more, 13 types or more or 14 types of genes selected from the group consisting of Gafa3, Lmf1, Fam13a, Gkn1, Gpr112, Sult1c4, Slc35f4, Gstt1, Gpr27, Pdia2, RIIAD1, GSTT1, SLC2A2 and HTATSF1P2, each are 5 times or more, 10 times or more, 20 times or more, 30 times or more, 40 times or more, 50 times or more, 60 times or more, 70 times or more, 80 times or more, 90 times or more, 95 times or more, or 100 times or more as low as other groups of cells, may be selected and used as the dental pulp stem cell to be used in a graft material for treating nerve damage according to the specification.

### [Method for treating nerve damage]

The present invention includes a method for treating nerve damage, including a step of grafting the graft material for treating nerve damage as mentioned above to an area of nerve damage.

The graft material for treating nerve damage can be injected into an area of nerve damage by e.g., a syringe. Alternatively, the area of nerve damage is dissected and then the graft material may be disposed. In the case where the graft material contains a xenogeneic cell, an immune suppressant such as cyclosporine can be administered together. As long as a nerve damage therapeutic effect can be obtained, the grafting material can be used in combination with other medicinal drugs.

The dose and administration times can be appropriately determined by those skilled in the art.

The subject to which the method for treating a nerve damage is to be applied is not limited to humans and may be other mammals (for example, mice, rats, rabbits, dogs, cats, monkeys, sheep, cows, horses).

In the specification, an example of a method for treating human brain infarction will be described below; however, the treatment method is not limited to the following example.

The graft material for treatment produced by the method for producing a graft material for treating nerve damage according to the present invention or the graft material for treating nerve damage according to the present invention, more specifically, dental pulp stem cells, are intravenously administered by use of a syringe pump from a peripheral vein at an injection rate of 2 mL/minute to a patient with human brain infarction, in an effective amount.

### [Kit for producing graft material for treating nerve damage]

The present invention includes a kit for producing a graft material for treating nerve damage. The kit contains a medium for culturing a dental pulp stem cell or all or part of components of the medium and FGF2. As the medium for culturing a dental pulp stem cell, a base medium or a medium for culturing a mesenchymal stem cell is mentioned. FGF2 and the medium may be separately contained or may be mixed together from the beginning. Furthermore, under the assumption that ultra-pure water, which is a material regularly stocked in laboratories, can be prepared by the user, all or part of requisite components for a medium may be contained so as to prepare the medium of the present invention only by adding the components to the water.

The kit of the present invention may be used in experiments performed in laboratories or used in a large scale culture. The kit may contain, other than a culture solution, e.g., a culture container, a virus filter, a coating material for a culture container, various reagents, a buffer and an instruction booklet.

The disclosures of all Patent Literatures and Non Patent Literatures cited in the specification are incorporated herein in their entirety by reference.

### Examples

Now, the present invention will be more specifically described based on Examples; however, the present invention is not limited to these. Those skilled in the art can modify the invention in various ways without departure from the significance of the present invention and such modifications are included in the range of the present invention.

### Example 1. Effect 1 of difference in culture method of dental pulp stem cell upon motor function recovery effect of model with total amputation of spinal cord

### 1. Experimental method

### 1-1. Animal and Material

Wistar rats (7 weeks old, female) were purchased from Japan SLC and an anesthetic drug, somnopentyl, was purchased from Kyoritsuseiyaku Corporation. Prior to animal experiments, a protocol of animal experiment was prepared in accordance with a predetermined format based on the regulation for safety and welfare of animal experiments and approval by the animal breeding/animal experiment committee of Gifu Pharmaceutical University was obtained.

### 1-2. Cell culture

From the evulsion tooth excised out, dental pulp stem cells were induced and proliferated in culture in accordance with the previous report (Tamaoki et al., J Dent Res. 2010 89: 773-778). The dental pulp stem cells successively cultured up to the 8th generation were sub-cultured in MSCGM medium (LONZA) 2 to 5 times to prepare cells (DP310) and sub-cultured in α-MEM medium (Sigma) containing 10 ng/ mL FGF2 and 10%FCS, 5 or 6 times to prepare dental pulp stem cells (DP31F).

### 1-3. Experimental method using total amputation model

### Preparation of model with total amputation of spinal cord

To Wistar rats (7 weeks old, female), somnopentyl was intraperitoneally administered in a dose of 40 mg/kg body weight. After anesthesia, the back was dissected along the midline at the position of the 10th thoracic spine in a length of 2 cm. The fat and muscle tissues were removed to expose the spine. The vertebral arch was removed and the 10th thoracic spine (T10) was dissected cross-sectionally with a sharp knife. After arrest of bleeding, the cultured dental pulp stem cells, which were suspended in each medium so as to contain 10⁶ cells/10 µL, were injected to the space between the rostral cut-end and the caudal cut-end of the cleavage site. Thereafter, the muscles of the back and the skin were sutured. After the surgery, it was confirmed that the hind limb at the same side of the cleaved spinal cord was paralyzed. The rats were raised in a routine manner and subjected to experiments. Note that cyclosporine serving as an immune suppressant was intraperitoneally injected in a dose of 10 mg/kg, every day.

### 2. Results

Recovery of motor function was evaluated based on BBB score (Basso DM et al., J Neurotrauma. 1995 Feb; 12 (1) : 1-21) (Figure 1).

Two weeks later, even an individual to a damaged part of which the cells were not injected but PBS or a culture supernatant alone was injected, recovered to the extent that one or two joints of the hind limb completely paralyzed slightly moved. However, no more recovery of motor function was observed in 7 weeks after the damage (BBB score = 1).

In contrast, in the group having DP310 grafted, as shown in Figure 1, three weeks after the damage, two joints became sufficiently movable in a half number of the individuals (7 out of 14). Four weeks after the damage, all individuals of the group showed significantly high motor function compared to the control group (BBB score = 3.5). In the group having DP31F grafted, one week after the damage, one joint of the hind limb became slightly movable. On and after two weeks, the individuals showed significantly high motor function compared to the control group and the DP310 grafted group (final BBB score = 6.5). The half of them (7 out of 13) was recovered to the extent that the body weight was supported by the paralyzed limb.

### Example 2. Effect 2 of difference in culture method of dental pulp stem cell upon motor function recovery effect of model with total amputation of spinal cord

### 1. Experimental method

### 1-1. Animal and Material

Animals were prepared in the same manner as in Example 1.

### 1-2. Cell culture

From the evulsion tooth excised out, dental pulp stem cells were induced and proliferated in culture in accordance with the previous report (Tamaoki et al., J Dent Res. 2010 89: 773-778). The dental pulp stem cells successively cultured up to the 12nd generation in MSCBM medium (LONZA) were sub-cultured in α-MEM medium (Sigma) containing 10%FCS, 7 or 8 times to prepare dental pulp stem cells (DP31S) and sub-cultured in α-MEM medium containing 10 ng/ mL FGF2 and 10%FCS, 7 or 8 times to prepare dental pulp stem cells (DP31F).

### 1-3. Experimental method using total amputation model Models with total amputation of spinal cord was prepared in the same manner as in Example 1.

### 2. Results

In the same manner as in Example 1, recovery of motor function was evaluated based on BBB score (Basso DM et al., J Neurotrauma. 1995 Feb; 12 (1): 1-21) (Figure 2).

In the group (control) where cells were not grafted and the group where dental pulp stem cells (DP31S) cultured in FGF2 free α-MEM medium containing 10% FSC were grafted, motor function recovery of the hind limb was rarely observed. Only one joint, in average, was slightly movable (BBS score = 1.9 ± 0.2, n = 45, BBS score = 1.9 ± 0.2, n = 14, respectively).

In contrast, in the group where dental pulp stem cells (DP31F) cultured in FGF2 containing α-MEM medium containing 10% FSC, were grafted, significant recovery effect of motor function was observed. In average, all three joints became movable (BBS score = 5.0 ± 0.7, n = 28).

### Example 3. Effect of difference in donor of dental pulp stem cell upon motor function recovery effect of model with total amputation of spinal cord

### 1. Experimental method

### 1-1. Animal and Material

Animals were prepared in the same manner as in Example 1.

### 1-2. Cell culture

From evulsion teeth excised out from three different donors (DP31, DP74, and DP264), dental pulp stem cells were induced, and proliferated in culture in accordance with the previous report (Tamaoki et al., J Dent Res. 2010 89: 773-778). The dental pulp stem cells derived from three donors each successively cultured 7 or 8 times in α-MEM medium containing 10 ng/ mL FGF2 and 10%FCS to prepare dental pulp stem cells (DP31F, DP74F, and DP264F) derived from three donors.

### 1-3. Experimental method using total amputation model In Models with total amputation of spinal cord were prepared in the same manner as in Example 1.

### 2. Results

Recovery of motor function was evaluated based on BBB score (Basso DM et al., J Neurotrauma. 1995 Feb; 12 (1): 1-21) in the same manner as in Example 1 (Figure 3).

In the DP264F grafted group, compared to DP31F and DP74F grafted groups (BBB score = 4.1 ± 0.7, n = 12), no recovery effect of motor function was observed (BBB score = 1.1 ± 0.2, n = 14).

### Experimental Example 1. Effect of difference in culture method of dental pulp stem cell upon expression of differentiation marker of nervous system cell

### 1. Experimental method

The rats of Example 1 in which dental pulp stem cells DP310 were grafted and the rats of Example 2 in which dental pulp stem cells DP31F were grafted, each were subjected to transcardial perfusion fixation with 0.1 M phosphate buffer (pH7.3) containing 4% paraformaldehyde, 7 weeks after grafting and a spinal cord tissue was excised out.

The spinal cord tissue excised out was soaked in a 20% sucrose solution in accordance with a conventional method, embedded in an OCT compound and sliced into thin sections by a cryostat. The thin sections were attached on slide glasses, soaked in Tris-HCl (pH7.4) containing 0.3% Triron X100 (registered trade mark) to enhance cell membrane permeability with an antibody, blocked with PBS containing 2% blockace (DS Pharma Biomedical Co., Ltd.) at room temperature for 30 minutes. Immunostaining was performed with a primary antibody such as anti-Human Nuclear antigen antibody (Millipore (MAB1281)), anti-Tuj1 antibody (Cell Signaling technology, #5568), anti-myelin basic protein (MBP) antibody (Millipore (AB980)), anti-CNPase antibody (Sigma (C5922)), anti-glial fibrillary acidic protein (GFAP) antibody (Dako (Z0334)), anti-growth asscciated protein 43 (GAP43) antibody (Chemicon (MAB347)) or anti-green fluorescence protein (GFP) antibody (Chemicon (AB3080)).

Human Nuclear antigen is a marker for human cells; CNPase is a marker for immature oligodendrocytes; and GFAP is a marker for immature astrocyte. Note that the graft cells were designed to express a GFP gene by use of a retroviral vector.

### 2. Results

The grafted dental pulp stem cells were identified based on GFP or Human Nuclear antigen positive. In DP310 before grafting, all markers (more specifically, Tuj1 (immature nerve cell marker), GFAP, CNPase, and Nestin (stem cell marker)) observed in immature nervous system cells, expressed; whereas, in DP310 in the spinal cord tissue, almost all cells were negative to Tuj1 and GFAP and positive to CNPase. In contrast in DP31F in the spinal cord tissue, a predetermined ratio of cells were positive to Tuj1 and MBP and almost all cells were negative to GFAP (it was not confirmed whether the cells positive to Tuj1 and positive to MBP are the same or not).

Thus, the possibility that grafted DP310 may be differentiated into oligodendrocytes and DP31F into a cell population containing nerve cells and oligodendrocytes, was suggested.

From the above, it was suggested that the following features were added to the dental pulp stem cells treated with FGF2, more specifically, to the cells cultured in MSCGM.
(i) When grafted in damaged spinal cord, the cells are changed into cells specifically differentiated into nerve cells.
(ii) Differentiation potency is limited (more specifically, the cells are specifically differentiated into nerve cells); however, proliferation potency is maintained.

These features indicate that the dental pulp stem cells treated with FGF2 are useful for treating nerve damage.

### Experimental Example 2. Global gene expression analysis

### 1. Experimental method

The dental pulp stem cells derived from two donors (DP31, and DP264) were cultured in MSCBM medium. Total RNA was extracted from the above dental pulp stem cells by RNeasy Plus Mini kit (Qiagen). After the RNA was quantified by Agilent 2100 Bioanalyzer (Agilent Technologies), 250 mg of RNA was taken and subjected to reverse transcription to obtain cDNA, which was amplified and labeled with Cy3-labeled CTP, by use of Low Input Quick Amp Labeling kit (Agilent Technologies) in accordance with the instruction booklet attached thereto. After the cDNA was purified, the cDNA was quantified by use of ND-1000 Spectrophotometer (Nano Drop Technologies) and allowed to hybridize to Whole Human Genome 4 x 44K oligo-DNA microarray (Agilent Technologies). After the hybridization, the array was continuously washed with Gene Expression Wash Pack (Agilent Technologies). The fluorescent image of the hybridized array was prepared by Agilent DNA Microarray Scanner (Agilent Technologies) and the fluorescent intensity was analyzed by Agilent Feature Extraction software ver.10.7.3.1. (Agilent Technologies). Analysis was made once with respect to each sample. The level of gene expression was analyzed by Gene Spring GX11.5 (Agilent Technologies).

### 2. Results

The genes of DP264 whose expression levels were 5 times or more as high as those in DP31 were listed in Table 1 and the genes of DP264 whose expression levels were 5 times or more as low as those in DP31 were listed in Table 2.

## Claims

1. A method for producing a graft material for treating nerve damage, comprising
a step of culturing a dental pulp stem cell in a medium substantially containing no growth factors except FGF2.

2. The method according to Claim 1, wherein the medium substantially containing no growth factors except FGF2 is a serum-containing base medium supplemented with FGF2 alone as a growth factor.

3. The method according to Claim 2, wherein the serum in the medium has a concentration of less than 15 wt%.

4. The method according to Claim 1, wherein the medium substantially containing no growth factors except FGF2 is a commercially available medium for culturing mesenchymal stem cells supplemented with FGF2 alone as a growth factor.

5. The method according to any one of Claims 1 to 4, wherein FGF2 in the medium has a concentration of 5 ng/mL or more.

6. The method according to Claim 5, wherein FGF2 in the medium has a concentration of 7 ng/mL or more.

7. The method according to any one of Claims 1 to 6, wherein the dental pulp stem cell used is a dental pulp stem cell, in which the expression level of 10% or more of genes in the group of genes listed in Table 1 is 5 times or more as high as an average expression level of the genes in dental pulp stem cells.

8. The method according to any one of Claims 1 to 7, wherein the dental pulp stem cell used is a dental pulp stem cell, in which the expression level of at least one gene selected from the group consisting of MYO1G RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as an average expression level of genes in dental pulp stem cells.

9. The method according to any one of Claims 1 to 8, wherein the dental pulp stem cell used is a dental pulp stem cell, in which the expression level of 10% or more of genes in the group of genes listed in Table 2 is 5 times or more as low as an average expression level of the genes in dental pulp stem cells.

10. The method according to any one of Claims 1 to 9, wherein the dental pulp stem cell used is a dental pulp stem cell, in which the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as an average expression level of genes in dental pulp stem cells.

11. The method according to any one of Claims 1 to 6, wherein among two or more groups of dental pulp stem cells, a group of dental pulp stem cells in which the expression level of 10% or more of genes in the group of genes listed in Table 1 is 5 times or more as high as other groups of cells, is selected as the dental pulp stem cell to be used.

12. The method according to any one of Claims 1 to 6 and 11, wherein among two or more groups of dental pulp stem cells, a dental pulp stem cell in which the expression level of at least one gene selected from the group consisting of MYO1G RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as other groups of cells, is selected as the dental pulp stem cell to be used.

13. The method according to any one of Claims 1 to 6, and 11 and 12, wherein among two or more groups of dental pulp stem cells, a dental pulp stem cell, in which the expression level of 10% or more of genes in the group of genes listed in Table 2 is 5 times or more as low as other groups of cells, is selected as the dental pulp stem cell to be used.

14. The method according to any one of Claims 1 to 6 and 11 to 13, wherein among two or more groups of dental pulp stem cells, a dental pulp stem cell, in which the expression level of at least one gene selected from the group consisting of Gafa3, Lmf1, Fam13a, Gkn1, Gpr112, Sult1c4, Slc35f4, Gstt1, Gpr27, Pdia2, RIIAD1, GSTT1, SLC2A2 and HTATSF1P2 is 5 times or more as low as other groups of cells, is selected as the dental pulp stem cell to be used.

15. The method according to any one of Claims 1 to 14, wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes.

16. A graft material for treating nerve damage, comprising a dental pulp stem cell and a medium substantially containing no growth factors except FGF2.

17. The graft material for treating nerve damage according to Claim 16, wherein the medium substantially containing no growth factors except FGF2 is a serum-containing base medium supplemented with FGF2 alone as a growth factor.

18. The graft material for treating nerve damage according to Claim 17, wherein the medium substantially containing no growth factors except FGF2 is a commercially available medium for culturing mesenchymal stem cells supplemented with FGF2 alone as a growth factor.

19. The graft material for treating nerve damage according to any one of Claims 16 to 18, wherein the dental pulp stem cell is a dental pulp stem cell, in which the expression level of 10% or more of genes in the group of genes listed in Table 1 is 5 times or more as high as an average expression level of genes of dental pulp stem cells.

20. The graft material for treating nerve damage according to any one of Claims 16 to 19, wherein the dental pulp stem cell used is a dental pulp stem cell, in which the expression level of at least one gene selected from the group consisting of MYO1G RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as an average expression level of genes of dental pulp stem cells.

21. The graft material for treating nerve damage according to any one of Claims 16 to 20, wherein the dental pulp stem cell used is a dental pulp stem cell, in which the expression level of 10% or more of genes in the group of genes listed in Table 2 is 5 times or more as low as an average expression level of genes of dental pulp stem cells.

22. The graft material for treating nerve damage according to any one of Claims 16 to 21, wherein the dental pulp stem cell used is a dental pulp stem cell, in which the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as an average of dental pulp stem cells.

23. The graft material for treating nerve damage according to any one of Claims 16 to 22, wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes.

24. A method for treating nerve damage, comprising a step of grafting a graft material for treating nerve damage produced by the method according any one of Claims 1 to 15 or the grafting material for treatment according to any one of Claims 16 to 23 to an area of nerve damage.

25. The method according to Claim 24, wherein the nerve damage is spinal cord injury, cerebral infarction, intracerebral hemorrhage, subarachnoid hemorrhage, spinal hemorrhage, compression injury of nerve caused by disk herniation, sciatic nerve pain or peripheral nerve damage caused by diabetes.

26. A kit for producing a graft material for treating nerve damage, comprising a medium and FGF2.

27. A method for selecting a material for a graft material for treating nerve damage from a plurality of groups of dental pulp stem cells, comprising selecting a dental pulp stem cell having at least one of the following properties (i) to (iv):
(i) the expression level of 10% or more of genes in the group of genes listed in Table 1 is 5 times or more as high as other groups of cells;
(ii) the expression level of at least one gene selected from the group consisting of MYO1G RBMY2FP, FILIP1, C1orf64, TNFRSF8, C2orf48, AGTR1, Dydc2, Znf708, Dct, Slc15a1, Zhddc22, Adam20, Gnao1, Csn2, Semg2, Dnah1, Ctag1a, Lrrc19, Lipg and Cbln2 is 5 times or more as high as other groups of cells;
(iii) the expression level of 10% or more of genes in the group of genes listed in Table 2 is 5 times or more as low as other groups of cells; and
(iv) the expression level of at least one gene selected from the group consisting of Gafa3, Lmf1, Fam13a, Gkn1, Gpr112, Sultlc4, Slc35f4, Gstt1, Gpr27, Pdia2, RIIAD1, GSTT1, SLC2A2 and HTATSF1P2 is 5 times or more as low as other groups of cells.
